(19) Europäisches Patentamt
European Patent Office
Office européen des brevets

(11) Numéro de publication: **0 386 045 B1**

(12) # FASCICULE DE BREVET EUROPEEN

(45) Date de publication de fascicule du brevet: **29.12.93**

(51) Int. Cl.[5]: **C07K 5/06**, C07K 7/06, A61K 39/385, A61K 37/02, //C12P21/04

(21) Numéro de dépôt: **88909281.3**

(22) Date de dépôt: **21.10.88**

(86) Numéro de dépôt internationale :
**PCT/BE88/00029**

(87) Numéro de publication internationale :
**WO 89/03843 (05.05.89 89/10)**

Le dossier contient des informations techniques présentées postérieurement au dépôt de la demande et ne figurant pas dans le présent fascicule.

(54) **DERIVES DE SYNERGIMYCINES DE TYPES A ET B.**

(30) Priorité: **22.10.87 BE 8701199**

(43) Date de publication de la demande:
**12.09.90 Bulletin 90/37**

(45) Mention de la délivrance du brevet:
**29.12.93 Bulletin 93/52**

(84) Etats contractants désignés:
**AT BE CH DE FR GB IT LI LU NL SE**

(56) Documents cités:
**EP-A- 0 117 934**

**The Merck Index, edition 10, 1983, Merck & Co., Inc., (Rahway, N.J., US), see page 1432, abstract No. 9810, "Virginiamycin"**

**Polymers in Medicine, volume 57, 1984, Springer - Verlag, (Berlin, DE), R. Duncan et al.: "Soluble synthetic polymers as potential drug carriers", pages 51-101**

(73) Titulaire: **ANDA BIOLOGICALS**
**37, rue de la Course**
**F-67000 Strasbourg(FR)**

(72) Inventeur: **Cocito, Carlo**
**Rue de l'Elevage 26**
**B-1340 Bruyères/Ottignies(BE)**
Inventeur: **Di Giambattista, Mario**
**Avenue de Mai 28**
**B-1200 Bruxelles(BE)**
Inventeur: **Pecher ,André**
**Av. Télémaque 8, Bte 10**
**B-1190 Bruxelles(BE)**

(74) Mandataire: **Van Malderen, Michel et al**
**p.a. Office van Malderen**
**avenue J.S. Bach 22/43**
**B-1080 Bruxelles (BE)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

Objet de l'invention

La présente invention est relative à des dérivés de la virginiamycine S et des dérivés de la virginiamycine M. Elle concerne également un procédé pour la préparation de ces dérivés. En outre, on décrit également les utilisations desdits dérivés en médecine vétérinaire et/ou humaine et pour la production d'anticorps spécifiques.

Résumé de l'état de la technique

Les virginiamycines S et M font partie d'une famille d'antibiotiques globalement connus sous les dénominations de "synergistines", "syneraimycines" et "streptogramines". Les antibiotiques de ce type, produits par les streptomycètes, contiennent deux types de substances, A et B en mélange, ayant une activité synergique in vivo. Les composés de type A issus de différents organismes se ressemblent entre eux, tout comme se ressemblent ceux de type B; toutefois, il n'y a aucune ressemblance entre les formules des composants A et B.

De nombreuses publications ont été faites sur ces antibiotiques et de nombreux produits existent sur le marché sous différentes dénominations commerciales (voir : The Merck Index, edition 10, 1983, Merck & Co., Inc. (Rahway, N.J., US) voir page 1432, abrégé no. 9810 "Virginiamycin".

On connaît aussi des techniques de production de modification antigénique de polypeptides capables de provoquer la formation d'anticorps (voir : EP, A, 0117934 (THE OHIO STATE UNIVERSITY) 12 septembre 1984 - pages 1 à 7.

Cependant, ce document ne mentionne pas l'application à la virginiamycine des techniques décrites et partant, les propriétés particulièrement recherchées du produit selon l'invention.

Dans le document: "Polymers in Medecine", vol. 56 (1984) Springer-Verlag Berlin, pp. 72-76, on décrit de manière générale la technique de ciblage de produits pharmaceutiquement actifs en les liant à des transporteurs spécifiques. A titre d'exemple, on mentionne les liaisons entre des restes de sucre et des anticorps.

**Inconvénients des produits connus**

Toutefois, malgré le fait que le mélange de S et M ait une activité supérieure à celle de la plupart des antibiotiques, l'emploi des virginiamycines en médecine est limité à cause de leur faible solubilité en milieux aqueux: en effet, le niveau d'assimilation à partir de l'intestin est très faible, et le coefficient de partage entre sang et tissus est négligeable.

Buts visés par la présente invention

Le but de la présente invention consiste à fournir des dérivés des produits naturels du type des dérivés de virginiamycine S et M.

Un autre but de la présente invention consiste à fournir des dérivés du type susmentionné qui présentent une solubilité améliorée en milieu aqueux et qui, de ce fait, atteignent des niveaux plus élevés dans le sang et les tissus.

Un but complémentaire consiste à fournir des dérivés de virginiamycine S et M qui exercent une action thérapeutique marquée dans le cas de maladies microbiennes, qui résulte de leurs propriétés intrinsèques.

Selon un autre aspect, la présente invention vise à fournir un procédé pour la préparation desdits dérivés à partir des produits naturels obtenus grâce aux streptomycètes appropriés.

Selon un aspect complémentaire, la présente invention porte sur des utilisations desdits antibiotiques en médecine humaine et/ou vétérinaire et pour la production d'anticorps spécifiques.

Description des principes de l'invention

Les dérivés de virginiamycine conformes à la présente invention sont choisis parmi le groupe constitué par les composés répondant à la formule générale

Z - X - R

dans laquelle Z représente un radical de virginiamycine de type S ou de type M, lié par sa position carbonyle réactive, par l'intermédiaire d'un bras X du type =N- ou =N-O-, à un substituant R représentant un atome d'hydrogène, un groupe alkyle, un groupe Alkyl-COOH, un groupe -alkyl(α-$NH_2$)COOH, un groupe

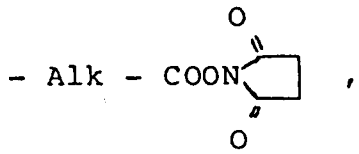

un groupe

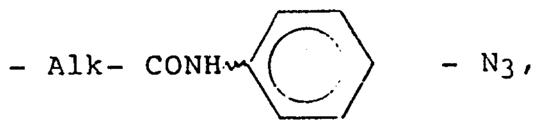

un groupe $\{$Alk-CONH$)_n$∿Prot ou un groupe $\{$Alk CONH$)_n$∿Rib, n étant fonction du nombre de $NH_2$ présents sur la protéine couplante ainsi que les sels, esters et éventuellement produits d'addition d'acides pharmaceutiquement acceptables.

L'invention concerne plus particulièrement des dérivés de Virginiamycine du type défini ci-dessus dans lesquels Z représente le radical de virginiamycine M, répondant à la formule

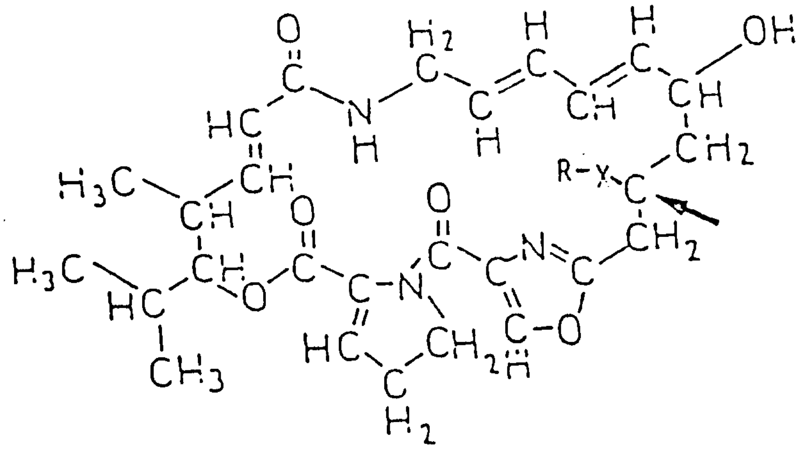

et le radical de virginiamycine S, répondant à la formule

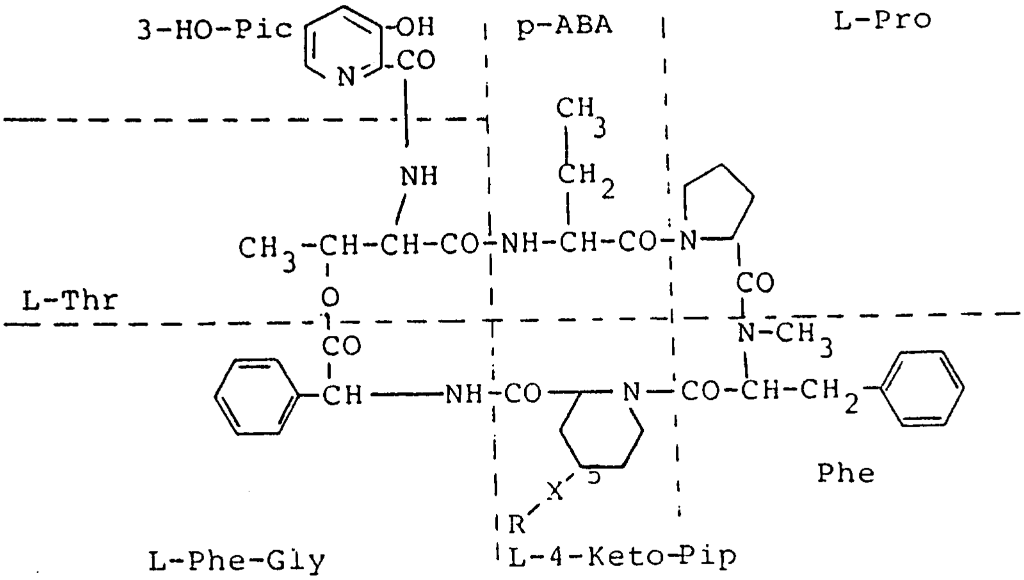

X et R ayant les significations définies précédemment.

Il a été constaté que les dérivés de l'invention sont biologiquement actifs et présentent un coefficient, de partition eau/solvants organiques plus avantageux que les produits de départ.

Selon la présente invention, on préfère les dérivés du type Z = N-O-R qui présentent une stabilité accrue en milieu aqueux.

Il y a encore lieu de noter que l'activité des antibiotiques ainsi obtenus peut être accrue sur un organe cible préalablement choisi en fonction des substituants R greffés sur le produit naturel par l'intermédiaire du bras X.

Selon un autre aspect de la présente invention, on obtient les dérivés définis ci-dessus en faisant réagir un composé du type $NH_2$-R ou $NH_2$-O-R, dans lequel R représente un atome d'hydrogène, un groupe alkyle linéaire ou ramifié, un groupe alk-COOH ou alkyl ($\alpha$-$NH_2$) COOH avec le ou les composés de départ produit(s) par des streptomycètes, à une température comprise entre 20 °C et 30 °C de préférence à une température de l'ordre de 20 °C, pendant 1 à 12 heures, de préférence pendant environ 4 heures, en milieu pyridine.

On peut ensuite faire réagir le produit ainsi obtenu avec un composé du type

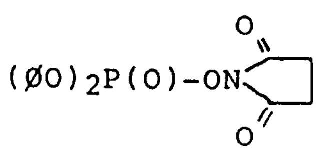

à une température comprise entre 10 °C et 30 °C, de préférence de l'ordre de 20 °C, pendant 12 à 48 heures, de préférence pendant 24 heures, en présence de triéthylamine.

Le produit ainsi obtenu convient particulièrement bien pour lier une virginiamycine à une protéine ou un ribosome.

On peut également le faire réagir avec tout composé possédant une fonction $-NH_2$.

Les dérivés selon la présente invention peuvent être utilisés individuellement ou en combinaison l'un avec l'autre, par exemple un dérivé du type A et un dérivé du type B, pour la préparation de compositions pharmaceutiques, éventuellement en combinaison avec d'autres principes actifs compatibles et des substances de support, des excipients et/ou solvants pharmacologiquement acceptables.

Les dérivés conformes à la présente invention comportant un radical protéique sont avantageusement utilisés pour produire des anticorps spécifiquement dressés contre l'antibiotique. Ceci permet donc d'utiliser de tels composés pour le diagnostic et/ou la détermination de la présence et le dosage de l'antibiotique, notamment par dosage enzymique.

Exemples d'exécution de l'invention

L'invention est décrite plus en détail à l'appui des exemples qui suivent.

Les solvants utilisés sont de qualité analytique.

Les chromatographies sur couche mince sont réalisées sur silicagel 60F254 d'épaisseur égale à 0,2 mm (aluminium) (Merck).

EXEMPLE 1

Synthèse du dérivé N-hydroxysuccinimido de la virginiamycine S (VS ESTER).

Cette synthèse se fait en trois étapes: 1) la synthèse du réactif SDPP; 2) le dérivé carpoxylique de VS et 3) l'ester activé de VS.

A. METHODE

1)

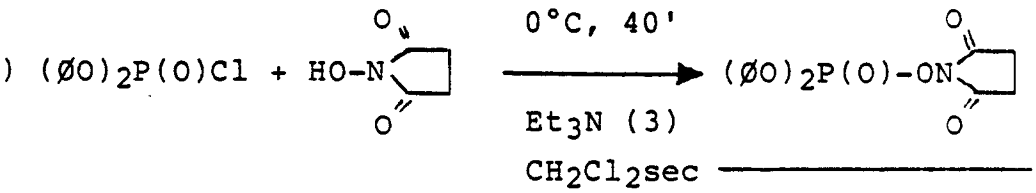


(1) (2) (4)

REACTION

A 0,01 mole de (2) dans 6 ml de $CH_2Cl_2$ sec (tamis moléculaire 4 Å + $P_2O_5$) sont ajoutés lentement sous agitation et dans l'ordre et à 0 °C: 0,01 mole de (3) et 0,01 mole de (1) dans 6 ml de $CH_2Cl_2$ sec, l'agitation est poursuivie encore durant 15 minutes à 0 °C. Le $CH_2Cl_2$ est évaporé à sec sous pression réduite à 40 °C. Le résidu est traité alors avec 50 ml d'éther donnant lieu à un précipité blanc. Celui-ci est filtré et lavé avec 20 ml d'éther, séché et dissout dans 100 ml d'éthylacétate. La phase organique est lavée trois fois avec 10 ml d'$H_2O$ à 0 °C et séchée sur tamis moléculaire 4 Å. L'acétate d'éthyle est évaporé à sec sous pression réduite à 40 °C. Le produit cristallisé est obtenu avec un rendement de 90%.

B. CARACTERISATION

Chromatographie sur couche mince réalisée sur silicagel 60F254 d'épaisseur égale à 0,2 mm (aluminium) (Merck)

| | | | | | |
|---|---|---|---|---|---|
| | | | | | RF |
| $CHCl_3$ / MeOH / HAC | (1) | DPCl | : | 0,05 |
| 95 : 4 : 4 | (2) | NHS | : | 0,165 |
| | (4) | SDPP | : | 0,57 |

La synthèse du SDPP (composé 4) et sa caractérisation sont décrites dans la littérature (Ogura H. et al., Tetrahedron Letters, 1980, 21, 1467-1468).

2)

$$(VS)C_5 = 0 + NH_2OCH_2COOH \xrightarrow[\text{pyridine (7) EtOH}]{20°C,\ 4\ hrs} (VS)C_5{=}NOCH_2COOH$$

(5) (6) (8)

C. REACTION

A 0,001 mole de (5) dans 20 ml d'éthanol sont ajoutés dans l'ordre: 250 µl de pyridine puis lentement 0,004 mole de (6) dans 20 ml d'éthanol. L'agitation se poursuit durant 4 heures à température ambiante.

Le solvant est évaporé à sec sous pression réduite à 40 °C. Le résidu est dilué alors dans 25 ml d'HCl 0,25 N. La phase aqueuse est extraite avec 4 fois 25 ml de chloroforme. La phase organique est lavée avec quatre fois 25 ml d'$H_2O$ distillée, puis séchée sur tamis moléculaire 4 Å. Le chloroforme est finalement évaporé à sec sous pression réduite à 40 °C. Le produit cristallisé est obtenu avec un rendement de 80%.

D. CARACTERISATION

Chromatographie sur couche mince

| | | |
|---|---|---|
| $CHCl_3$ / MeOH / HAC | (5) $(VS)C_5 = 0$ | : 0,62 |
| 95 : 4 : 4 | (8) VSCOOH | : 0,32 |

$^1$H-RMN: 4.52-4.56 ppm (2H, α-COOH)

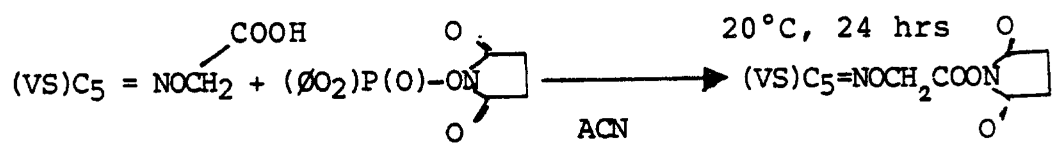


(8) (4) $Et_3N$ (3) (9)

E) REACTION

A 0,0001 mole de (8) dans 1 ml d'acétonitrile, sont ajoutés dans l'ordre et sous agitation: 0,0005 mole de (3) et lentement 0,0005 mole de (4) dans 2 ml d'acétonitrile.

L'agitation se poursuit durant 24 heures à température ambiante et à l'abri de la lumière.

Le produit est conservé à 4 °C, à l'abri de la lumière, dans l'acétonitrile.

F. CARACTERISATION

1) chromatographie sur couche mince

| | | RF |
|---|---|---|
| $CHCl_3$ / MeOH / HAC | (8) VSCOOH | : 0,32 |
| 95 : 4 : 4 | (4) SDPP | : 0,57 |
| | (9) VS ESTER | : 0,50 |

2) $^1$H RMN: 2.86 ppm (4H,O-succinimide)

EXEMPLE 2:

Couplage de la virginiamycine S à l'albumine bovine

L'albumine bovine (BSA) est une molécule qui possède environ 52 résidus lysine par mole (52 fonctions ε aminées) qui sont d'excellents sites réactionnels vis-à-vis de "l'ester activé" de la virginiamycine S.

L'albumine bovine utilisée présente une pureté de 99%.

METHODE

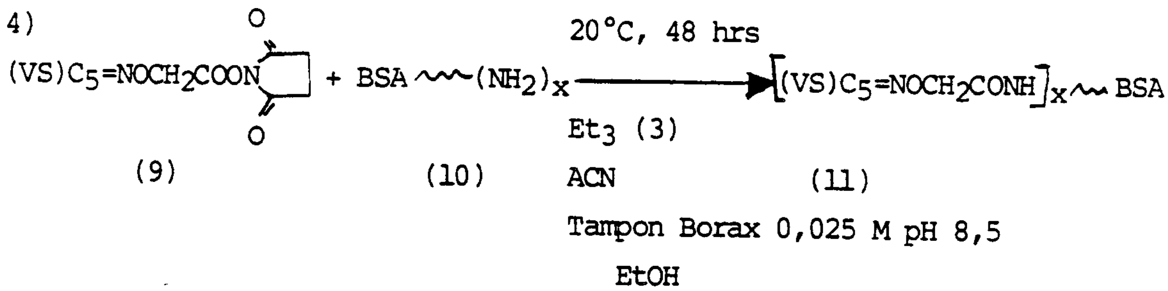

(9) (10) (11)

A. REACTION

A 1,43 µmole de (9) dans 100 ul d'acétonitrile sont ajoutés sous agitation et dans l'ordre: 2 µl de (3); 150 ul d'EtOH, 150 µl de tampon Borax 0,025 M pH 8,5; 100 ul d'une solution à 10 mg/ml de BSA (14,3 nmole) et 200 ul d'EtOH.

L'agitation se poursuit durant 48 heures à l'abri de la lumière, à température ambiante et sous $CaCl_2$.

B. PURIFICATION DE $(VS)_x$∿∿BSA (FIG.1)

A la fin de la réaction, le produit de couplage $(VS)_x$∿∿BSA est précipité à -70 °C durant 1 heure en ajoutant au milieu réactionnel 50 µl de KCl 2 M et 8 ml d'EtOH.

Après une centrifugation, le surnageant est éliminé et le culot séché.

Le culot est alors solubilisé dans 1 ml d'$H_2O$ distillée.

L'opération est répétée deux fois (précipitation, centrifugation, séchage du culot).

Le culot est alors solubilisé dans l ml d'$H_2O$ distillée et 40 µl d'urée 10 M.

Cet échantillon est déposé en tête de colonne Séphadex G150 et élué avec une solution $NaH_2PO_4$ 4,8 $10^{-2}$ M/$NH_4Cl$ 0,1 M.

Des fractions de 1 ml sont récoltées et celles présentant une absorbance $A_{260}$ sont concentrées à ± 1 ml sur tapis de PEG. L'échantillon est alors dialysé durant 12 heures à 4 °C contre 500 ml de NaCl 0,1 N.

Le dialysat est finalement lyophilysé et conservé à -20 °C.

C. EVALUATION DU RENDEMENT DE COUPLAGE

1) Le lyophilysat est solubilisé dans 1 ml d'$H_2O$ distillée, le dosage en protéines totales (BSA) est réalisé par la méthode de Lowry (24) en utilisant une calibration standard en BSA entre 4 et 20 µg/ml.

2) A l'aide d'un spectrofluorimètre Aminco-Bowman SPF Ratio II: λ Excitation de 330 nm et λ Emission de 420 nm; la fluorescence (u.a) de l'échantillon est mesurée et comparée dans les mêmes conditions d'analyse, à la fluorescence d'un échantillon de virginiamycine S de concentration connue.

La concentration totale en virginiamycine S dans l'échantillon en est déduite.

3) Le rendement du couplage est alors calculé comme suit:

$$\text{Rdt} = \frac{\text{Concentration totale en VS}}{\text{Concentration totale en BSA}} = \frac{\text{mole VS}}{\text{mole BSA}}$$

4) Une étude du rendement de couplage en fonction du rapport

$$\frac{[\text{VSE}]}{[\text{BSA}]}$$

mis en jeu au début de la réaction a été réalisée (Fig.1).
Ce rendement varie entre 5 et 35 moles de VS/mole de BSA. Dans les conditions de réaction décrites en A, un rendement de 15 ± 3 moles de VS/mole de BSA est généralement obtenu.

D. CARACTERISATION DE $(VS)_{15}$∿BSA

1) Chromatographie sur couche mince

$CHCl_3$ / MeOH / HAC
95 : 4 : 4

| | RF | FLUO |
|---|---|---|
| (10) BSA: | 0 | - |
| (9) VS ESTER: | 0,5 | + |
| (11) $(VS)_{15}$∿BSA: | 0 | + |

2) Chromatographie sur colonne Séphadex G150 (Fig. 2)

a) L'échantillon standard de virginiamycine S est récupéré dans les fractions entre 30 et 70 ml.
b) L'échantillon standard de BSA (4 mg/ml) est récupéré dans les mêmes conditions entre 10 et 30 ml. Aucune fluorescence n'est détectée dans ce cas.
c) L'échantillon purifié en $(VS)_{15}$∿BSA (4 mg/ml) est récupéré dans les mêmes conditions entre 10 et 30 ml. Une fluorescence est détectée dans ce cas.
Cette analyse nous permet de mettre en évidence la stabilité de $(VS)_{15}$∿BSA (il a résisté à toutes les étapes de la purification).

3) Electrophorèse en gel de SDS-polyacrylamide (Fig. 3)

a) Diverses protéines de masses moléculaires connues (dont la BSA) sont séparées sur un gel de SDS-polyacrylamide (25). Plus une protéine migre avec un Rf important, plus petite est sa masse moléculaire.
b) $(VS)_{15}$∿BSA est soumis aux mêmes conditions d'analyse. Le Rf obtenu pour celui-ci permet d'estimer sa masse moléculaire (79.200).
c) Celle-ci, comparée à la masse moléculaire de BSA et tenant compte de la masse moléculaire de la virginiamycine S permet de déduire un rendement de couplage de 15 moles ± 3 de VS/mole de BSA ce qui confirme les résultats en C 4).

EXEMPLE 3:

Production d'anticorps spécifiquement dressés contre la virginiamycyne S.

A. IMMUNISATION

1) Les anticorps anti-VS sont générés par immunisation de deux lapins de race néo-zélandaise avec $(VS)_{15}$~~BSA.

2) Les injections sont effectuées par voie sous-cutanée selon le protocole suivant:

a) la première dose de 0,5 mg dans 500 µl d'$H_2O$ physiologique et 500µl d'adjuvant de Freund complet (10 injections de 100 µl).

b) au jour 8, une double dose est administrée, soit 1 mg dans 500 µl d'$H_2O$ physiologique et 500 µl d'adjuvant de Freund incomplet.

c) une nouvelle dose de 0,5 mg est administrée au jour 15 et 22 selon b).

3) Le sang est récolté par une veine de l'oreille

a) 20 ml, avant toute injection (sérum contrôle)

b) 50 ml, 7 jours après la deuxième injection

c) 50 ml, 7 jours après chaque rappel effectué toutes les 3 semaines.

4) Le sérum est obtenu par centrifugation du sang à 3000 rpm durant 5 min. Le surnageant est prélevé. Le sérum est conservé à -20°C.

B. EXTRACTION DES IgG TOTALES DU SERUM

1) Une colonne de DEAE affi-gel blue (Bio-Rad) est préparée à cet effet. Un volume de 7 ml de support est utilisé pour purifier un ml de sérum.

2) Cette colonne est équilibrée avec 2 volumes d'un tampon A, soit: 0,02 M Tris.HCl pH 8,0; 0,028 M NaCl; 0,02% $NaN_3$.

3) Le sérum est dialysé vis-à-vis du tampon A.

4) Le dialysat est déposé en tête de colonne et élué avec 3 volumes de tampon A. Des fractions de 1 à 2 ml sont collectées et analysées au spectrophotomètre à 280 nm. Les fractions montrant une absorbance sont réunies et concentrées à ± 1 ml sur tapis de PEG;

5) La colonne est alors régénérée avec 2 volumes d'une solution de Guanidine.HCl 6 M ou NaSCN 6 M suivis de 2 volumes de tampon A.

C. PURIFICATION DES IgG SPECIFIQUES (ANTI-VS)

1) Couplage de la virginiamycine S à l'affi-gel 102

L'Affi-gel 102 (Bio-Rad) est un gel d'agarose portant des ramifications contenant une fonction aminée terminale. Selon spécification, il y a 15 umoles/ml gel de ces groupements aminés.

a) Structure

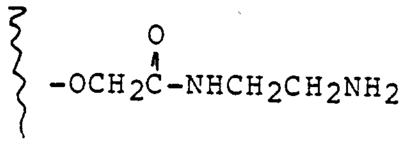

b) Schéma de réaction

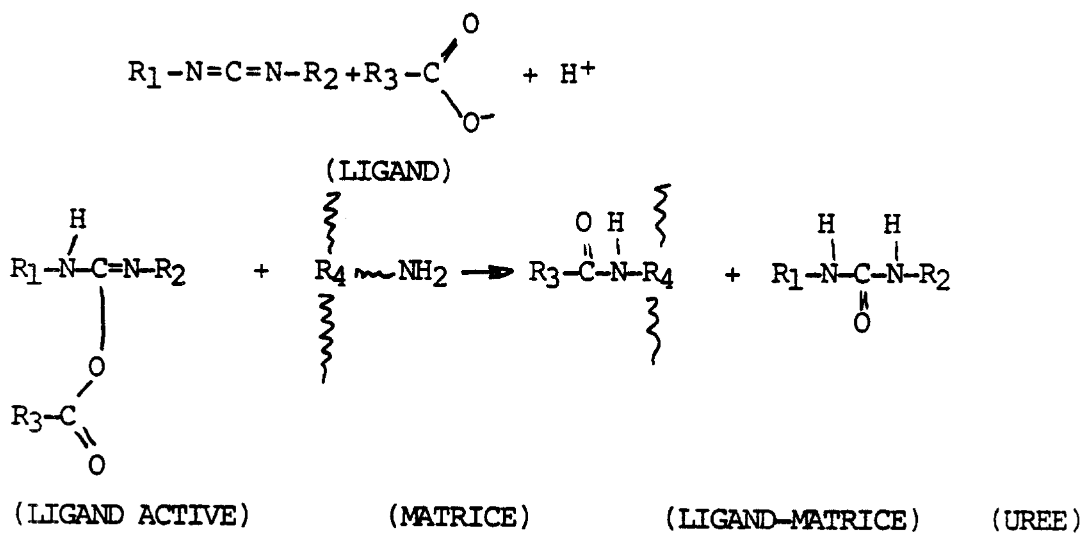


c) Réaction

1) 30 mg de VSCOOH sont dissous dans 5 ml d'EtOH et 1 ml d'$H_2O$ à pH 4,5 (HCl 0,1 N) (solution A).
2) 21 mg d'EDC sont dissous dans 2 ml d'$H_2O$ à ph 4,5 (HCl 0,1 N) (solution B).
3) La solution A est ajoutée à 10 ml d'affi-gel 102, sous agitation et à l'abri de la lumière.
Des fractions de 200 µl de la solution B sont alors additionnées toutes les 2 minutes. La réaction est poursuivie à température ambiante, sous agitation et à l'abri de la lumière durant 20 heures.

2) PREPARATION D'UNE COLONNE D'AFFINITE (AFFI-GEL 102 VS)

a) 5 ml du produit de la réaction décrite en 1)c sont déposés dans une colonne de 10 ml;
b) La colonne est lavée et conditionnée avec 50 ml d'EtOH/$H_2O$ (50:50), suivi de 40 ml de tampon A (B 2).

3) OBTENTION DES IgG SPECIFIQUES DE VS

a) La fraction concentrée en IgG totales, obtenue en B, est déposée en tête de colonne d'affinité et éluée avec le tampon A jusqu'à ce que les fractions récoltées montrent une valeur de densité optique négligeable (IgG non-spécifiques sont éliminées.
b) Les IgG dressées spécifiquement contre la virginiamycine S et qui sont absorbées sur la colonne sont décrochées à l'aide d'une solution de glycine.HCl 50 mM à pH 2,8.
Les fractions dont la densité optique est supérieure à 0 sont rassemblées et dialysées contre le tampon A (12 heures, 4°C). Les IgG spécifiques sont alors concentrées sur tapis de PEG.
c) La concentration en immunoglobulines G est estimée par la méthode de Bradford. Celles-ci sont conservées à -20°C.

D. CARACTERISATION DES IMMUNOGLOBULINES ANTI-VS

1) IMMUNODIFFUSION (OUCHTERLONY) (Fig.4)

a) Une solution de: 0,1 M NaCl; 0,05 M Tris-HCl pH 7,5; 0,1 mM EDTA; 1% agarose est déposée sous forme de film sur une plaque de verre.
b) A égale distance, on répartit 6 puits périphériques autour d'un puits central.
c) 15 ug d'anticorps anti-VS sont déposés dans le puits central et 7 à 22 µg du complexe $(VS)_{15}$ ∿∿ BSA et/ou $(VS)_8$ ∿∿ PA sont déposés dans les puits périphériques.
d) Après diffusion pendant 12 heures à température ambiante, la plaque est lavée à l'aide d'une solution de NaCl 0,9%, séchée et colorée au bleu de Coomassie (0,5% dans $MeOH/H_2O/HAC/CHCl_3$ - 42,5:45:10:2,5).

2) ELISA

a) A une plaque de microtitration (Flow Laboratories) comportant 96 puits est ajouté 0,5 mg de $(VS)_{15}$ ∿∿ BSA (Fig 5), ou de $(VS)_8$ ∿∿ PA (Fig. 6) ou de BSA seule. Cette opération est effectuée (à 4°C, 12 hres) en présence de 1% de glutaraldéhyde dans 0,15 M NaCl.
b) L'excès de complexe est éliminé.
c) La plaque est alors saturée (1 hre, à température ambiante) avec une solution à 1 mg/ml de lactalbumine dans un tampon B soit: 0,15 M NaCl, 0,05% (v/v) Tween 80: 0,15 M tampon phosphate pH 7,2.
d) La plaque est lavée 5 fois avec le tampon B.
e) 50 µl d'une série de dilutions d'IgG totaux ou d'anticorps anti-VS purifiés (dans 0,9% NaCl) sont ajoutés à chaque puits.
La plaque est incubée 1 heure à température ambiante.
f) La plaque est lavée cinq fois avec le tampon B.
g) 50 µl d'une dilution (1/800) d'anti-immunoglobulines de lapins marquée à la péroxydase sont ajoutés à chaque puits. La plaque est incubée une heure à température ambiante.
h) La plaque est à nouveau lavée avec du tampon B.
i) 50 µl d'une solution O,2% en orthophénylène diamine dans O,O15% $H_2O$; 15 mM ac-citrique; 6O mM d'un tampon phosphate pH 6,3 sont finalement additionnés à chaque puits et le tout incubé 30 minutes à 37°C à l'abri de la lumière.
j) La réaction est stoppée en ajoutant à chaque puits 50 µl d'$H_2SO_4$ 4,5 M.
k) La densité optique de chaque puits est mesurée à 492 nm à l'aide d'un spectrophotomètre pour plaques Elisa (Kontron Analytical SLT 210).

EXEMPLE 4

Marquage d'affinité des ribosomes 70s par la virginiamycine S "ester activé"

A. LIAISON SPECIFIQUE DE LA VIRGINIAMYCINE S "ESTER ACTIVE" AU RIBOSOME 70S

La virginiamycine S est fluorescente en solution acqueuse en présence d'ions bivalents tels que le $Mg^{++}$ ou le $Ca^{++}$. La modification (augmentation) du rendement de fluorescence de la virginiamycine S, suite à sa liaison au ribosome, est exploitée dans le but de mesurer la constante d'association du complexe formé, la stoechiométrie de la réaction ainsi que le synergisme induit par la virginiamycine M qui se traduit par une augmentation de la constante d'association de VS d'un facteur d'environ 6.

De plus, lorsque l'on incube le complexe ribosome-VS en présence d'érythromycine ou d'un autre macrolide, on peut suivre la cinétique de déplacement de la virginiamycine S du complexe (diminution de la fluorescence).

Ces deux types d'expériences réalisées avec la virginiamycine S "ester activé" montrent que celle-ci avait une constante d'association très semblable à la virginiamycine S (1,5 $10^6$ $M^{-1}$ pour 2,5 $10^6$ $M^{-1}$), que sa stoechiométrie était toujours de 1:1 et que l'érythromycine déplaçait aussi la virginiamycine S "ester activé". Cette réaction se fait dans une échelle de temps de l'ordre de la minute alors que la réaction covalente se fait sur une échelle de temps 10 à 20 fois plus grande.

Ces résultats indiquent que la virginiamycine S et le dérivé "ester activé" ont bien le même site de fixation sur le ribosome.

B. COUPLAGE SPECIFIQUE DE LA VIRGINIAMYCINE S AU RIBOSOME 70S

Celui-ci s'effectue à l'aide de "l'ester activé" de la virginiamycine S. En effet, un nombre important de groupements $NH_2$-lys et NH-his ribosomiaux sont susceptibles de réagir avec ce dérivé.

De plus, la probabilité d'avoir un résidu lysine ou histidine à proximité du site de fixation de VS est grande car ils représentent 10 à 20 % du nombre total de résidus dans chaque protéine ribosomiale.

1) Matériel

- Les ribosomes 70S d'E. coli sont obtenus par la méthode de Tissières: 1 densité optique (DO) de ribosomes 70S à 260 nm correspond à 24 pmoles.
- L'érytromycine utilisée est un produit Boehringer.

2) Méthode

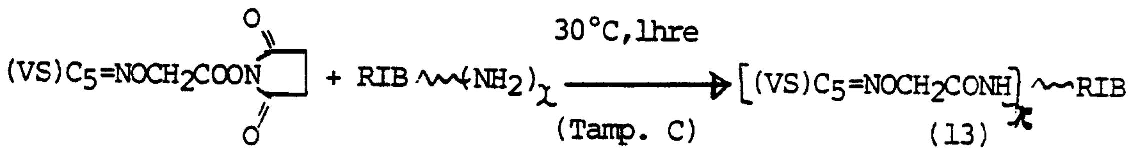


(9) (12) (13)

a) 5 nmoles de ribosomes 70S sont réactivées 5 minutes à 40 °C dans 1 ml de tampon C soit: 10 mM $MgCl_2$; 50mM TEA pH 7,8; 100 mM KCl et 4 % EtOH.

b) Réaction n° 1

5 nmoles de 70S réactivés sont traités avec 50 nmoles de VS ester. La formation du complexe est effectuée 5 minutes à 4 °C. La réaction est incubée à 30 °C durant 1 heure, à l'abri de la lumière puis stoppée par l'addition de 500 nmoles d'éthanolamine et incubée 15 minutes à température ambiante.

Réaction n° 2

5 nmoles de 70S réactivés sont traités avec 50 nmoles d'Ery (saturation du site de fixation de VS) 5 minutes à 4° C. 50nmoles de VS ester sont alors ajoutées et le mélange est incubé 5 minutes à 4 °C, puis à 30 °C durant 1 heure, à l'abri de la lumière. La réaction est stoppée comme décrit au n° 1.

Réaction n° 3

5 nmoles de 70S réactivés sont traités avec 50 nmoles de VS ester (préalablement hydrolysé 1 heure à 30 °C par 2500 nmoles d'éthanolamine) 5 minutes à 4 °C. Le mélange est ensuite incubé à 30 °C durant 1 heure, à l'abri de la lumière. La réaction est stoppée comme décrit au n° 1.

3) PURIFICATION DE RIB~~VS

Après chaque réaction, on ajoute 5 nmoles d'Ery de façon à déplacer l'excès de VS non lié de façon covalente au ribosome (10 minutes à 30 °C).

Chacun des trois échantillons est alors déposé (1, 2, 3) sur une colonne de Sépharose 4B (25 ml) et élué avec le tampon C.

Les fractions de 1 ml sont récoltées et mesurées à 260 nm. On mesure aussi la fluorescence (II, C, 2). Les fractions contenant les ribosomes (DO > 0, F > 0) sont rassemblées et précipitées par l'ajout de 0,7 volumes d'EtOH, centrifugées à 5000 rpm durant 10 minutes. Le surnageant est éliminé et le culot resuspendu dans 1 ml de tampon C (l'opération est répétée deux fois: précipitation, centrifugation).

Le marquage d'affinité des ribosomes par l'ester activé de la virginiamycine S est réalisé tel quel (panneau A) ou après saturation du site de fixation par l'érythromycine (panneau B). Le premier se réfère à un marquage total (une partie aspécifique + une partie spécifique), le second correspond à un marquage aspécifique uniquement.

Le panneau C représente un contrôle, dans lequel l'ester activé de la virginiamycine S est préalablement hydrolysé par l'éthanolamine.

Les mélanges réactionnels sont alors séparés par chromatographie sur une colonne de Sépharose 4B.

L'absorbance $A_{260nm}$ (-0-) et la fluorescence (-●-) sont mesurées dans chacune des fractions comme décrit en IV,B,3.

4) ESTIMATION DU RENDEMENT DE COUPLAGE (Fig. 8)

10 DO de ribosomes 70S purifiés (cela correspond à 240 pmoles) provenant de la réaction n° 1 et n° 2 sont portés à 1 ml avec le tampon C. Le spectre d'émission des deux échantillons est alors mesuré entre 370 et 520 nm à λ excitation de 330 nm. La fluorescence prouve la présence de VS dans l'échantillon. Les rendements de couplage obtenus sont de 1,5 moles de VS par mole de ribosome pour la réaction n° 1 (aspécifique + spécifique) et de 0,6 modes de VS par mode de ribosome pour la réaction n° 2 (aspécifique).

Après purification des échantillons provenant de deux types de marquage (aspécifique + spécifique) et (aspécifique seul), comme décrit pour la fig. 7, les fractions contenant les ribosomes sont rassemblées.

La quantité de VS présente à une concentration connue en ribosome est mesurée en réalisant un spectre d'émission de fluorescence (λ Ex 330).

La courbe (-0-) correspond au premier type de marquage (aspécifique + spécifique), la courbe (-●-) au deuxième type de marquage (aspécifique uniquement).

Par rapport à un standard de VS, les rendements sont estimés respectivement à 1,5 et 0,6 modes de VS liées par mode de ribosome.

5) EXTRACTION DES PROTEINES RIBOSOMIALES

Les protéines ribosomiales sont obtenues par extraction à l'acide acétique selon la méthode de Hardy et al.

6) ELISA (fig. 9)

L'Elisa est effectué selon la méthode décrite ci-dessus sur des ribosomes 70S contrôles et sur les protéines ribosomiales extraites de ceux-ci. Il est également réalisé sur des ribosomes 70S-VS obtenus après purification de la réaction n° 1 et sur les protéines ribosomiales extraites de ceux-ci.

Le dosage enzymatique (Elisa) est réalisé comme décrit dans la fig. 5.

Les protéines ribosomiales purifiées, provenant de 10 pmoles de ribosomes marquées par d'ester activé de la virginiamycine S (-▲-) ou contrôles non marquées (-△-) sont utilisés comme antigène.

Le graphique montre également la réaction possible des particules ribosomiales entières marquées (-●-) et des particules ribosomiales contrôles (-O-).

EXEMPLE 5

Le même schéma réactionnel explicité dans l'exemple 1 a été appliqué pour la synthèse d'un dérivé de VS portant une fonction (α-$NH_2$).COOH. Le réactif employé pour cette réaction, la canaline, a la structure

$$H_2N.(CH_2)_2.\underset{\displaystyle NH_2}{\underset{|}{CH}}.COOH.$$

Le produit obtenu

$$(VS)C_5{=}NO(CH_2)_2.\underset{\displaystyle NH_2}{\underset{|}{CH}}.COOH$$

porte un groupement basique et acide, et de ce fait peut être couplé à toute une série de réactifs pour les groupements -$NH_2$ et -COOH. Par exemple, la réaction de l'acide lactobionique avec de groupement -$NH_2$ du dérivé de VS susmentionné. Autre exemple, le couplage de la D-glucamine avec le -COOH de l'ester activé de VS. Ces deux types de dérivés ont des solubilités en milieu aqueux et des coefficients de partage tissulaires différents que ceux de VS.

Selon l'invention, en une seule étape (antibiotique + réactif), les demandeurs sont donc parvenus à greffer sur une portion de l'antibiotique spécifique, un bras possédant en bout de chaîne une fonction

chimique susceptible d'être modifiée dans un deuxième temps à l'aide de divers produits synthétiques ou naturels (acides aminés, sucres, protéines...) et pouvant de ce fait conduite à l'obtention d'une série de dérivés de l'antibiotique.

Il en découle les avantages suivants :

Les produits obtenus, au cours de la première étape (oximation de la fonction cétonique) sont stables en milieu aqueux et ce contrairement aux produits obtenus à l'aide de réactifs du type NH2-(CH2)n-X (amines).

On peut en outre facilement moduler la fonction en bout de chaîne en utilisant divers dérivés oxoaminés (NH2O-(CH2)n-NH2,NH2O-(CH2)n-COOH....).

Les rendements sont voisins de 100 % et les réactions parasites n'existent pour ainsi dire pas (<5%).

La fonction chimique de fin de chaîne peut également être modifiée aisément.

Les demandeurs ont indiqué dans leur demande la réaction de formation de l'ester-activé de la virginiamycine S ainsi que la réaction de ce dernier avec une macromolécule (BSA) afin de démontrer la facilité de la technique.

La réaction avec la BSA a été rpésentée à titre d'exemple et ne constitue cependant pas une fin en soi. De plus, la possibilité de préparer des anticorps spécifiques démontrent clairement la stabilité du lien entre l'antibiotique et la protéine porteuse.

A titre de comparaison, il convient de noter que l'utilisation de dérivés du type

NH2-(CH2)n-X avec X = COOH
= NH2
= OH

ou encore NH4Ac, NH2-NH2....
conduisent toujours à des mélanges de plusieurs produits ainsi qu'à des rendements n'excédant pas 60 %. Les produits ainsi obtenus se montrent généralement peu stables en milieu aqueux et régénèrent les réactifs de départ (hydrolyse-réaction équilibrée).

Légende de la Figure 4.

1. 22 µg $(VS)_{15}$ ∿∿ BSA
2. 11 µg $(VS)_{15}$ ∿∿ BSA
3. 7 µg $(VS)_{15}$ ∿∿ BSA
4. 22 µg BSA
5. 15 µg IGg anti-VS

## Revendications

**1.** Dérivés de virginiamycine répondant à la formule générale

Z - X - R

dans laquelle Z représente un radical de virginiamycine de type S ou de type M, lié par sa position carbonyle réactive, par l'intermédiaire d'un bras X du type =N- ou =N-O-, à un substituant R représentant un atome d'hydrogène, un groupe alkyle, un groupe alkyl-COOH, un groupe -alkyl-(α-$NH_2$)COOH, un groupe

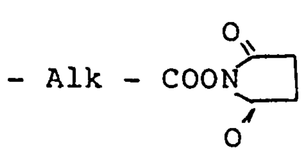 ,

un groupe

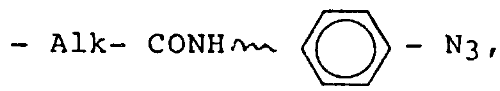

un groupe ⟨Alk-CONH)$_n$∿Prot ou un groupe ⟨Alk CONH)$_n$∿Rib, n étant fonction du nombre de -$NH_2$ présents sur la protéine couplante ainsi que les sels, esters et éventuellement produits d'addition d'acides pharmaceutiquement acceptables.

**2.** Dérivés de virginiamycine selon la revendication 1 dans lesquels Z représente le radical de virginiamycine M, répondant à la formule

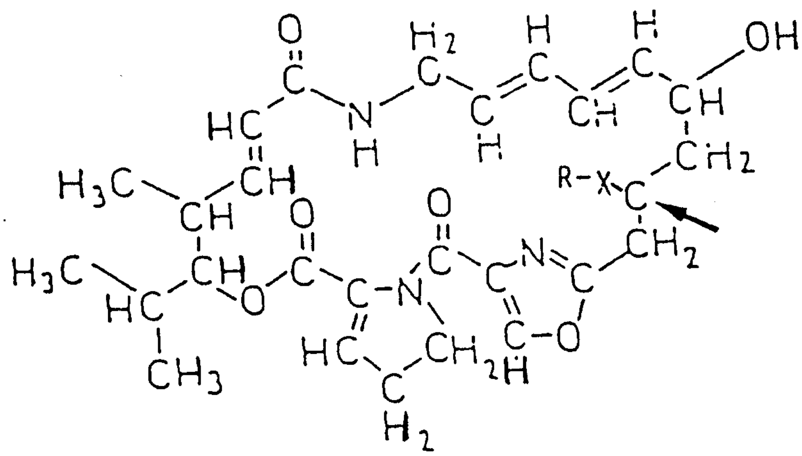

et le radical de virginiamycine S, repondant à la formule

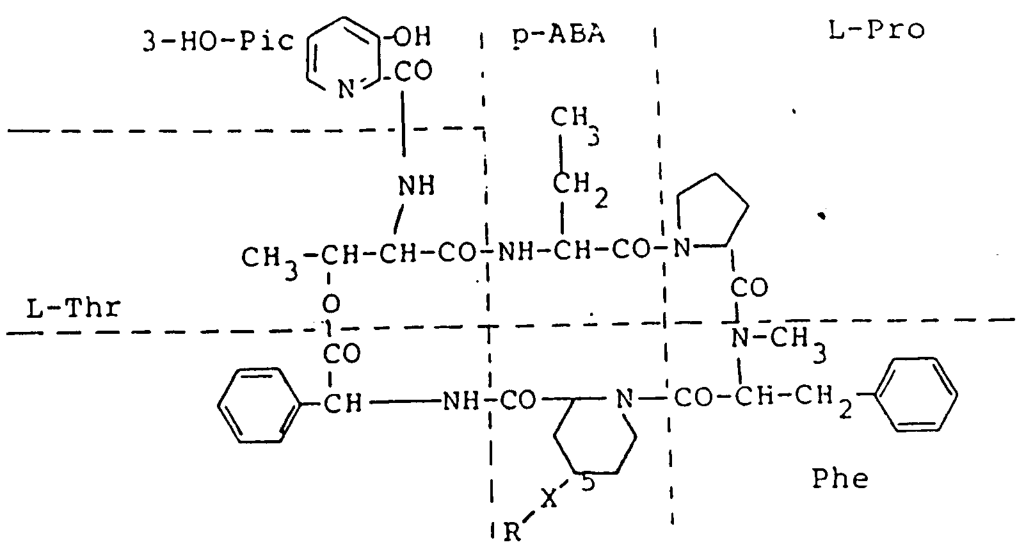

X et R ayant les significations définies précédemment.

**3.** Procédé de préparation des dérivés de virginiamycine selon les revendications 1 et 2 caractérisé en ce qu'on fait réagir un composé du type $NH_2$-R ou $NH_2$-O-R, dans lequel R représente un atome d'hydrogène, un groupe alkyle linéaire ou ramifié, un groupe alk-COOH avec le ou les composés de départ produit(s) par des streptomycètes, à une température comprise entre 20 °C et 30 °C, de préférence à une température de l'ordre de 20 °C, pendant 1 à 12 heures, de préférence pendant environ 4 heures, en milieu pyridine.

4. Procédé selon la revendication 2 caractérisé en ce qu'on fait réagir le produit obtenu avec un composé du type

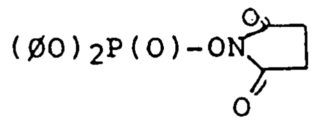

à une température comprise entre 10 ° C et 30 ° C, de préférence de l'ordre de 20 ° C, pendant 12 à 48 heures, de préférence pendant 24 heures, en présence de triéthylamine.

5. Procédé selon la revendication 4 caractérisé en ce qu'on fait réagir le produit obtenu avec tout composé pourvu d'une fonction -$NH_2$.

6. Préparation pharmaceutique contenant les dérivés selon l'une quelconque des revendications 1 ou 2 ou obtenus selon le procédé de l'une quelconque des revendications 3 à 5, individuellement ou en combinaison l'un avec l'autre, éventuellement en combinaison avec d'autres principes actifs compatibles et des substances de supports, excipients et/ou solvants pharmaceutiquement acceptables.

7. Utilisation des dérivés selon la revendication 1 comportant un radical protéique pour la production d'anticorps spécifiquement dresses contre l'antibiotique.

**Claims**

1. Derivatives of virginiamycin according to the formula

Z - X - R

where Z is a virginlamycin radical of type S or M which is linked through its reactive carbonyl, by means of an arm X of the type = N- or = N-O-, to a substituent R representing a hydrogene atom, an alkyl group, an alkyl-COOH group, a group -alkyl-($\alpha$-$NH_2$)COOH, a group

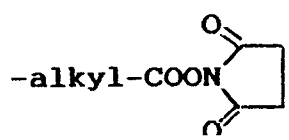 ,

a group

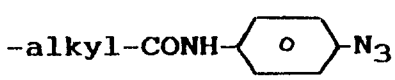 ,

a group -(Alk-CONH)$_n$∿Prot, or a group -(Alk-CONH)$_n$∿Rib, where n is a function of the -$NH_2$ content of the coupling protein as well as pharmaceutically acceptable salts, esters and optionally, acid addition compounds with pharmaceutically acceptable acids.

2. Derivatives of virginiamycin according to claim 1 wherein Z is the virginiamycin M radical according to the formula

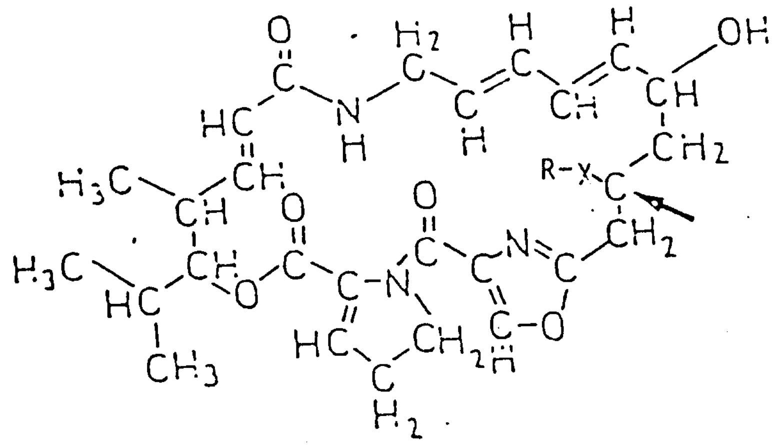

and the virginiamycin S radical, corresponding to the formula

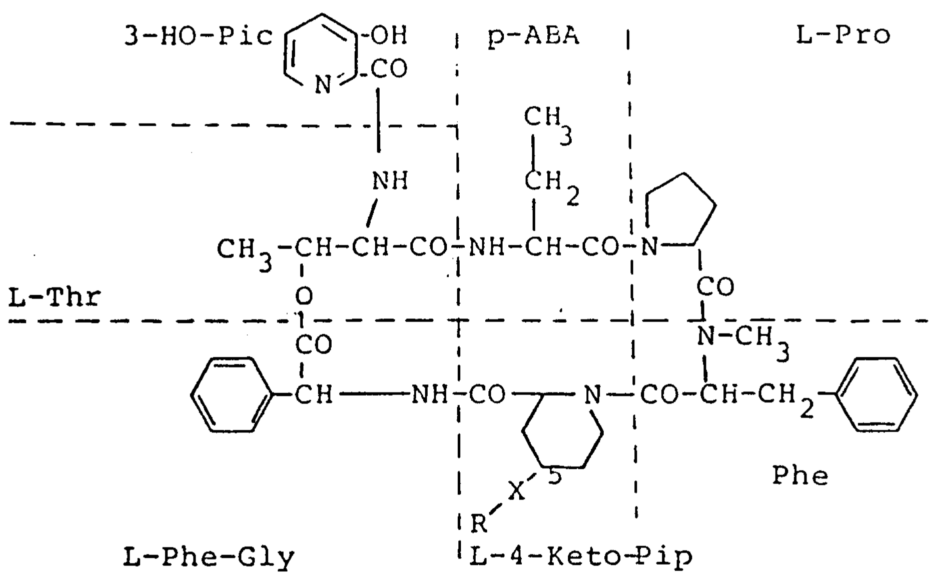

X and R being defined as here above.

**3.** Process for the preparation of the derivatives of virginiamycin according to claims 1 and 2, characterized in that a compound of the type $NH_2$-R or $NH_2$-O-R wherein R represents a hydrogen atom, a linear or branched alkyl group, an alkyl-COOH group is reacted with the starting product(s) obtained by streptomycetes at a temperature of 20°-30°C, preferably of about 20°C, for 1 to 2 hours, preferably 4 hours, in pyridine medium.

**4.** Process according to claim 3 characterized in that the product thus obtained is reacted with a compound of the type

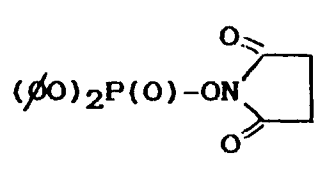

at a temperature of between 10° and 30°C, preferably about 20°C, for 12 to 48 hours, preferably for 24 hours in the presence of triethylamine.

**5.** Process according to claim 4 wherein the obtained product is reacted with any compound carrying a $-NH_2$ function.

**6.** Pharmaceutical preparation containing the derivatives according to any one of claims 1 or 2 or produced by the process of any of claims 3 to 5, either individually or in combination with one another, optionally in combination with other compatible pharmaceutically acceptable active principles, vehicules, excipients and/or solvents.

**7.** Use of the derivatives according to claim 1 carrying a protein radical for the production of specific antibodies directed against the antibiotic.

**Patentansprüche**

**1.** Virginiamycin-Derivate, die der allgemeinen Formel

Z - X - R

entsprechen, bei der Z ein Radikal von virginiamycin vom Typ S oder vom Typ M repräsentiert, das bei seiner reaktiven Carbonyl-Position über einen Arm X vom Typ =N oder =N-O an einen Substituenten R gebunden ist, der ein Wasserstoffatom, eine Alkylgruppe, eine Alkyl-COOH-Gruppe, eine -Alkyl-($\alpha$-$NH_2$)COOH-Gruppe, eine

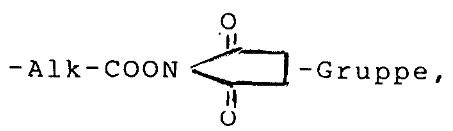

eine

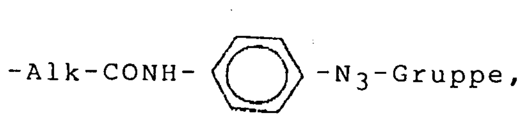

eine -(Alk-CONH)$_n$-Prot-Gruppe, oder eine -(Alk-CONH)$_n$-Rib-Gruppe repräsentiert, wobei n von der Anzahl der $-NH_2$ abhängt, die auf dem koppelnden Protein vorhanden sind, sowie die pharmazeutisch annehmbaren Salze, Ester und eventuellen Additionsverbindungen von Säuren.

**2.** Virginiamycin-Derivate gemäß Anspruch 1, bei denen Z das M-Virginiamycin-Radikal repräsentiert, das der Formel

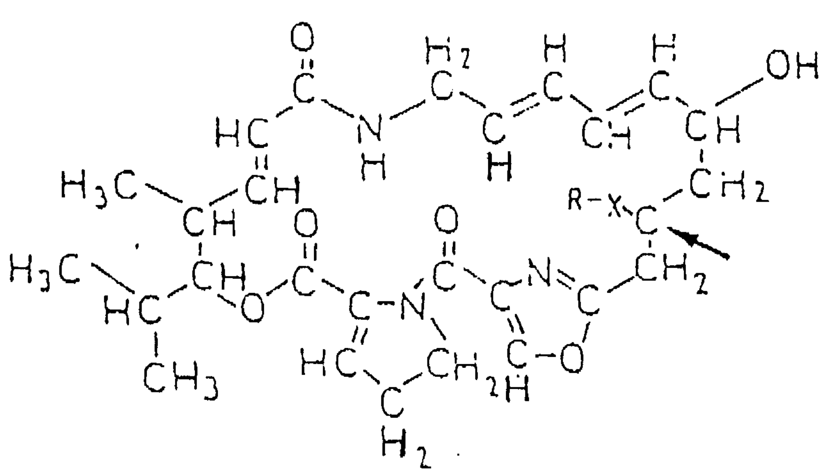

entspricht, und das S-Virginiamycin-Radikal repräsentiert, das der Formel

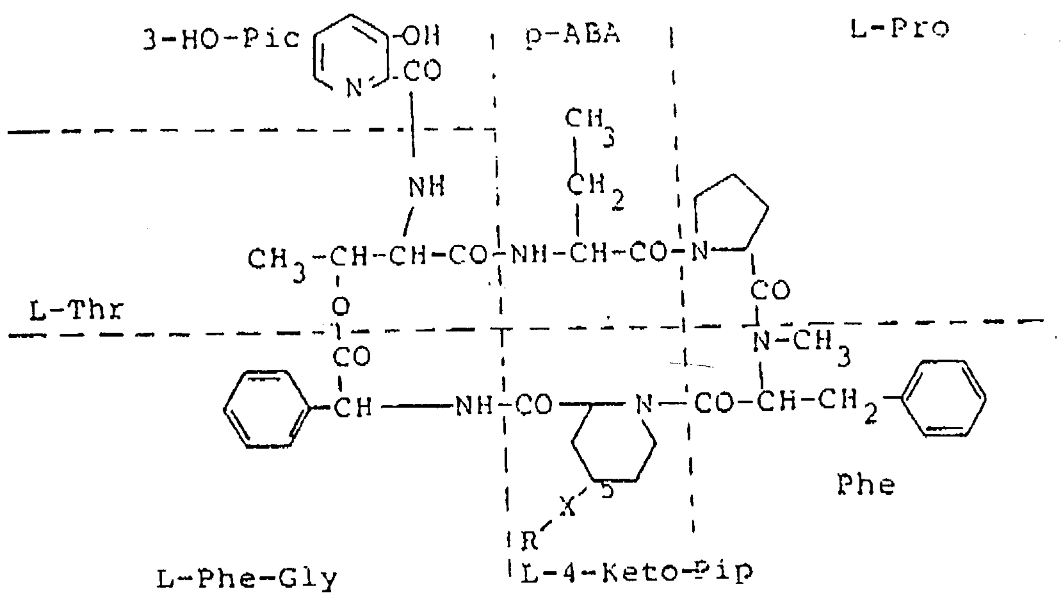

entspricht, wobei X und R die oben angegebenen Bedeutungen haben.

3. Verfahren zur Herstellung der Virginiamycin-Derivate gemäß den Ansprüchen 1 und 2, dadurch gekennzeichnet, daß man eine Verbindung vom Typ $NH_2$-R oder $NH_2$-O-R, bei der R ein Wasserstoffatom, eine lineare oder verzweigte Alkylgruppe, oder eine Alk-COOH-Gruppe repräsentiert, mit der oder den von Streptomyzeten erzeugten Ausgangsverbindungen bei einer Temperatur zwischen 20 °C und 30 °C während 1 bis 12 Stunden, vorzugsweise während ungefähr 4 Stunden, in einer Pyridin-Umgebung reagieren läßt.

4. Verfahren gemäß Anspruch 2, dadurch gekennzeichnet, daß man das erhaltene Produkt mit einer Verbindung vom Typ

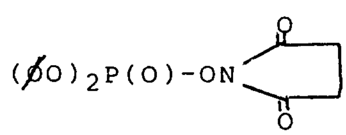

bei einer Temperatur zwischen 10 °C und 30 °C, vorzugsweise von ungefähr 20 °C, während 12 bis 48 Stunden, vorzugsweise während 24 Stunden, in Gegenwart von Triäthylamin reagieren läßt.

5. Verfahren gemäß Anspruch 4, dadurch gekennzeichnet, daß man das erhaltene Produkt mit jeder Verbindung, die mit einer $-NH_2$-Funktion versehen ist, reagieren läßt.

6. Pharmazeutisches Präparat, das die Derivate gemäß irgendeinem der Ansprüche 1 oder 2, oder die gemäß dem Verfahren von irgendeinem der Ansprüche 3 bis 5 erhaltenen Derivate, einzeln oder miteinander kombiniert, enthält, eventuell in Kombination mit anderen kompatiblen wirksamen Prinzipien und mit pharmazeutisch annehmbaren Trägersubstanzen, Exzipienten und/oder Lösungsmitteln.

7. Verwendung der Derivate gemäß Anspruch 1, die ein Protein-Radikal enthalten, zur Erzeugung von spezifisch gegen das Antibiotikum gerichteteten Antikörpern.

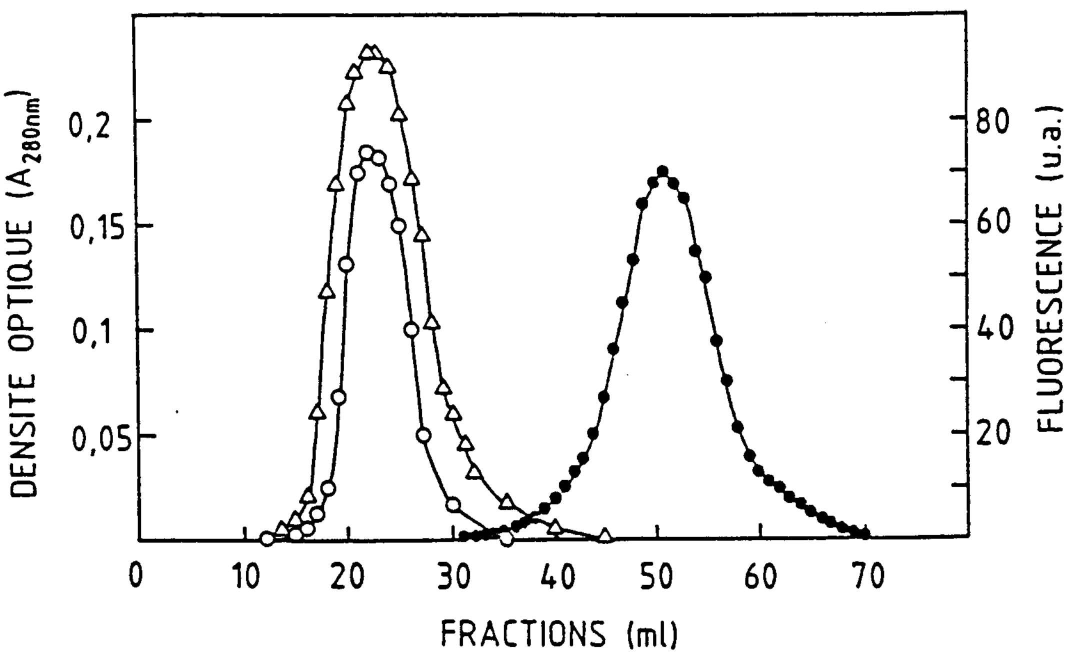
DENSITE OPTIQUE ($A_{280nm}$)
0,2
0,15
0,1
0,05
FLUORESCENCE (u.a.)
80
60
40
20
0
10
20
30
40
50
60
70
FRACTIONS (ml)

FIG.1

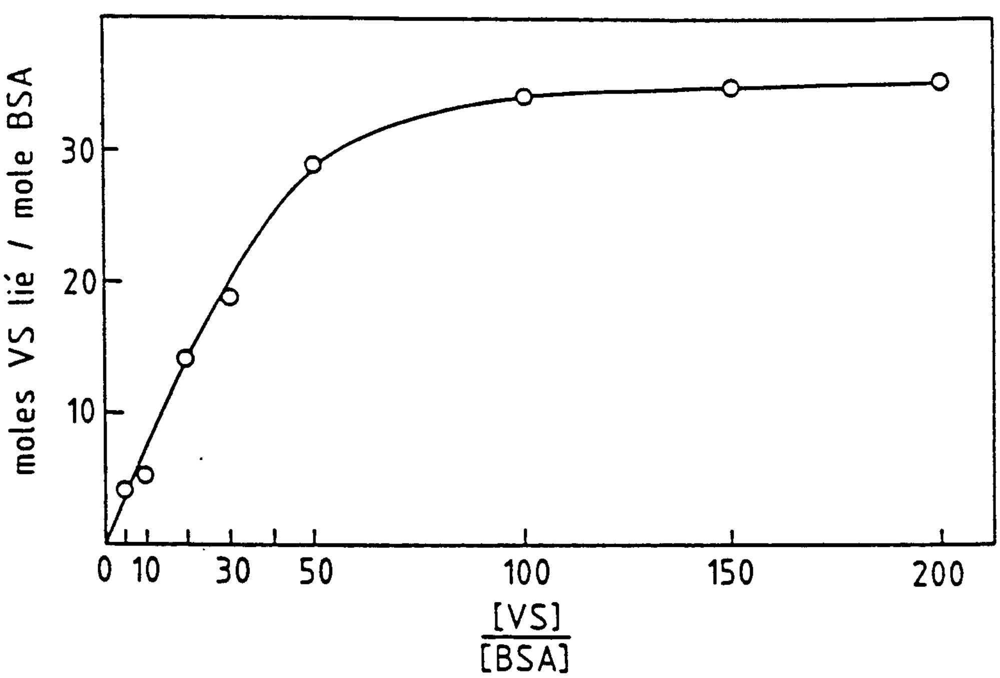
moles VS lié / mole BSA
30
20
10
0
10
30
50
100
150
200
[VS] / [BSA]

FIG.2

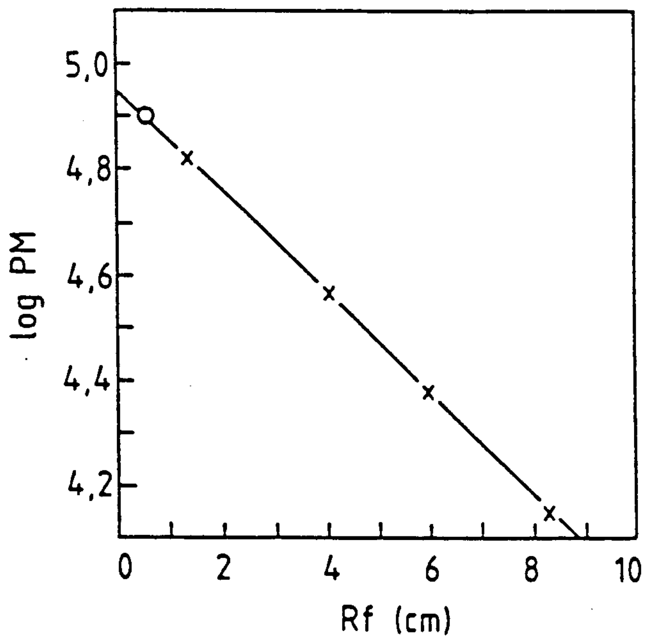


FIG.3

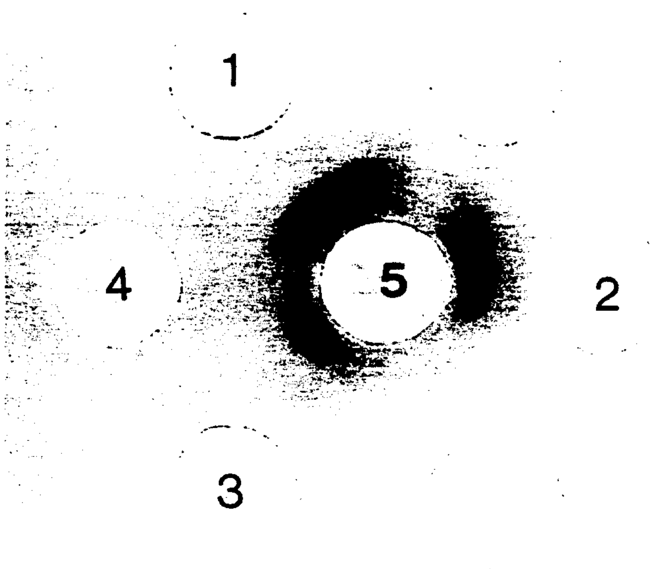


FIG. 4

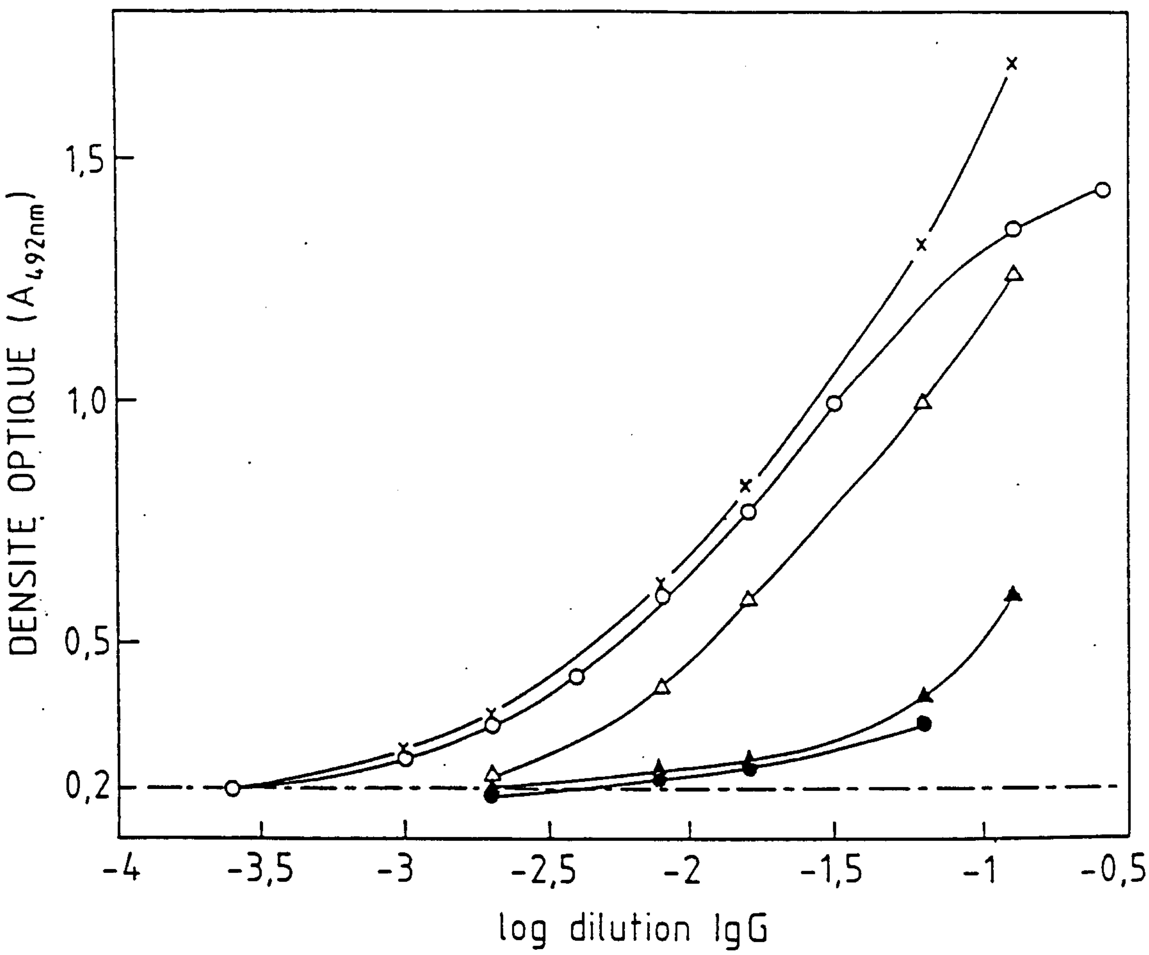
DENSITE OPTIQUE ($A_{492nm}$)
1,5
1,0
0,5
0,2
-4
-3,5
-3
-2,5
-2
-1,5
-1
-0,5
log dilution IgG

FIG.5

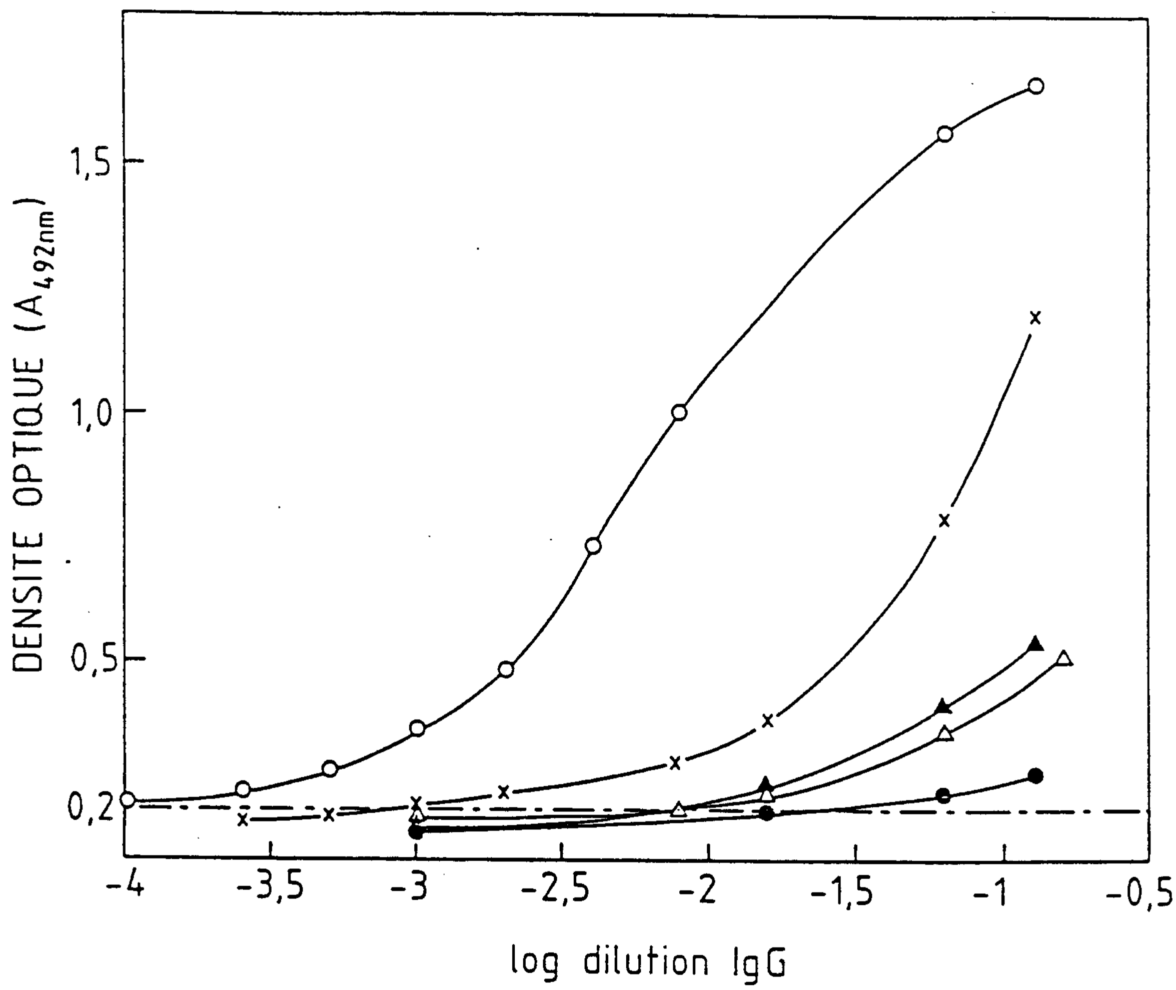
DENSITE OPTIQUE (A492nm)
1,5
1,0
0,5
0,2
-4
-3,5
-3
-2,5
-2
-1,5
-1
-0,5
log dilution IgG

FIG.6

FIG.7

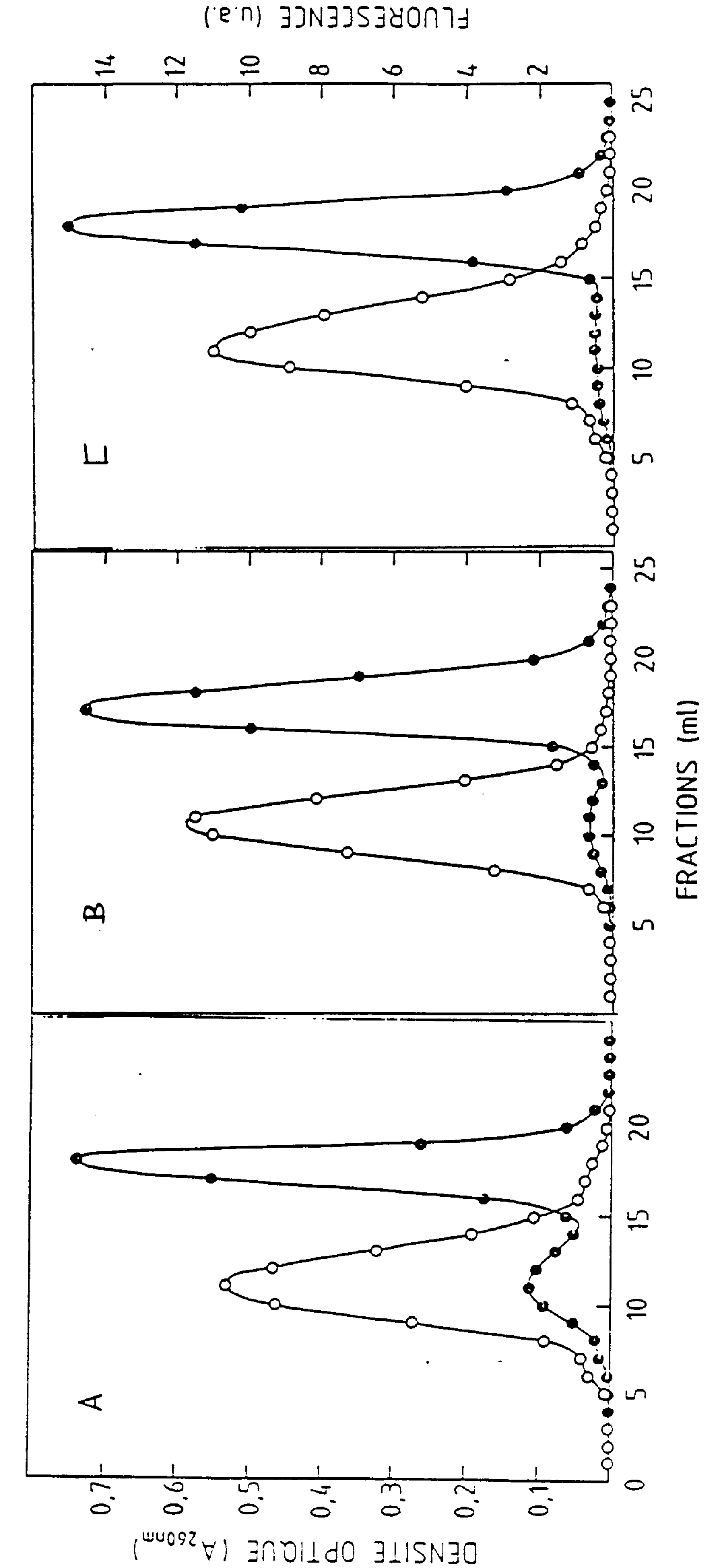
A
B
C
FLUORESCENCE (u.a.)
DENSITE OPTIQUE ($A_{260nm}$)
FRACTIONS (ml)
0
5
10
15
20
25
0,1
0,2
0,3
0,4
0,5
0,6
0,7
2
4
6
8
10
12
14

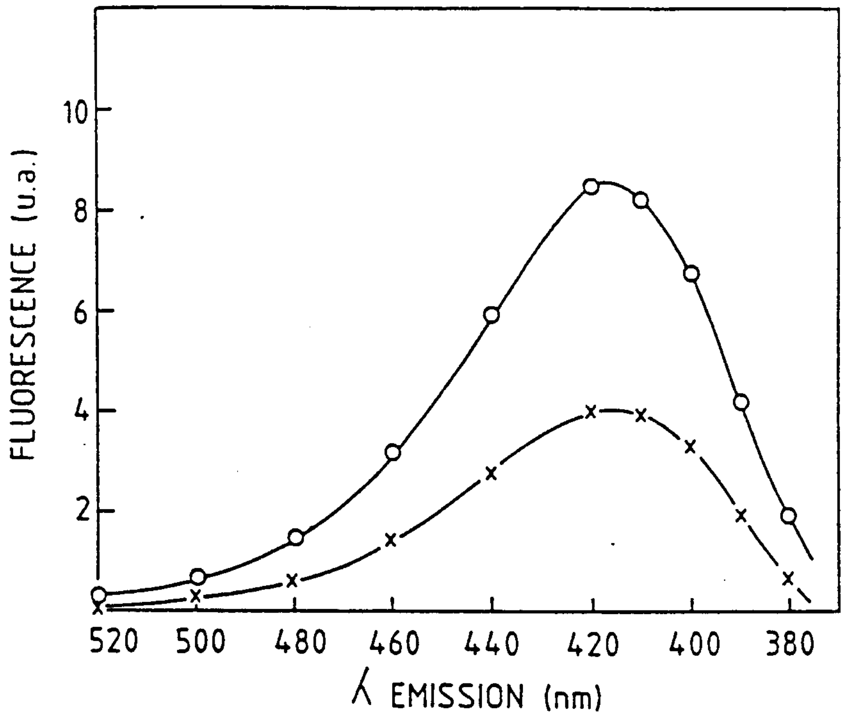
FLUORESCENCE (u.a.)
10
8
6
4
2
520 500 480 460 440 420 400 380
λ EMISSION (nm)

FIG.8

FIG.9

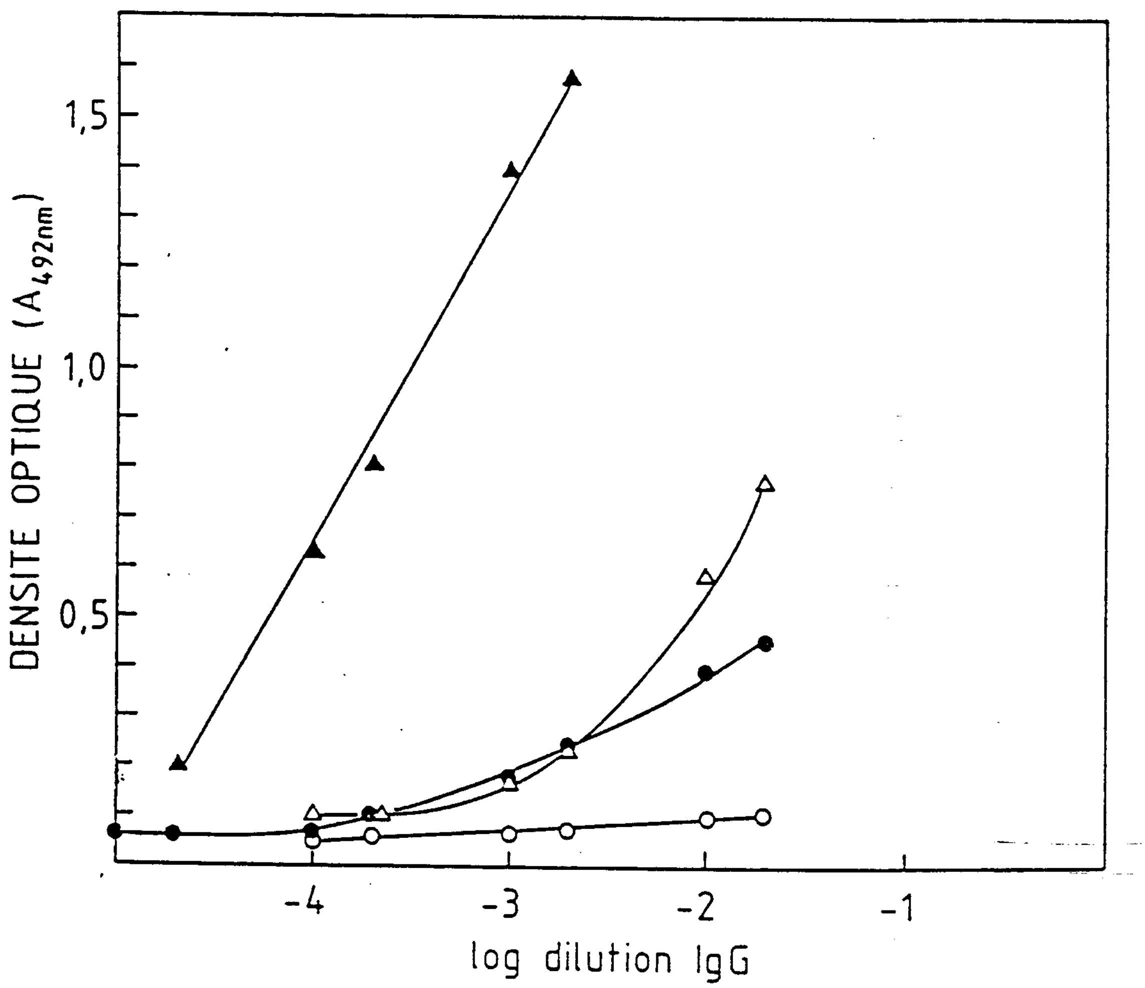
DENSITE OPTIQUE ($A_{492nm}$)
1,5
1,0
0,5
-4 -3 -2 -1
log dilution IgG